# EUROPEAN PATENT APPLICATION

(11) **EP 3 574 867 A1**
(43) Date of publication of application: **04.12.2019**
(21) Application number: 18745332.9
(22) Date of filing: 24.01.2018
(51) Int. Cl.: A61F 2/00

(54) **TISSUE COMPRESSION DEVICE**

(30) Priority: 24.01.2017 CN 201710054501
(71) Applicant: Shanghai Clinical Engine Technology Development Co., Ltd., Shanghai 200439 (CN)
(72) Inventor: SUN, Yinghao, Shanghai 200439 (CN)
(74) Representative: Viering, Jentschura & Partner mbB Patent- und Rechtsanwälte
(86) International application number: PCT/CN2018/073906
(87) International publication number: WO 2018/137634

(57) **Abstract**

A tissue compression device is disclosed. The tissue compression device includes a pump (301), a liquid storage chamber (1.5), catheters (2), and a compression device (3). The liquid storage chamber (1.5) is coupled to the pump (301), the liquid storage chamber (1.5) stores fluid of a preset volume, the liquid storage chamber (1.5) communicates with the compression device (3) via the catheters (2), the pump (301) provides power for fluid delivery between the liquid storage chamber (1.5) and the compression device (3), and the compression device (3) is used to nonlinearly compress a tissue wall relative to an axis of a tissue. The tissue compression device has a simple structure, occupies small space, is easy to be implanted, and has low power consumption and a simple operation.

## Description

### TECHNICAL FIELD

Embodiments of the present invention relate to the technical field of medical equipment, and particularly, to a tissue compression device.

### BACKGROUND

The male urination disorders, for example, urine leakage and even urinary incontinence, caused due to degenerative changes of tissues or organs, the traumatic injury of the pelvic floor, the surgical complication, and the like is one refractory disease that troubles many patients. Currently, one main method for clinically curing the male urinary incontinence is to implant a series of assemblies, including a urethra constraint device, a reservoir, a mechanical switch, a liquid flow connecting pipe, and the like, into a male patient. Because such type of implantation device has a complex structure, and leads to a large operative wound and a long postoperative recovery time, and the switch needs to be manually pressed, tissue fibrosis finally is formed on a surface of the reservoir, performance of the reservoir is deteriorated, and a use effect of a prosthesis is affected.

Another serious disadvantage is that an element that compresses, clamps, or limits a body organ possibly may injure a urethra tissue wall. In this way, a compression action of the element on the organ causes that the element may invade the organ with time, and even may penetrate a wall of the compressed organ. In addition, blood circulation in the tissue wall of the compressed organ finally is blocked due to pressure exerted by the element, and as a result, the blood circulation is poor or the blood circulation stops, deteriorating the compressed tissue.

In view of all factors above, currently, there is no ideal therapeutic device for male urine leakage or urinary incontinence.

### SUMMARY

In view of the foregoing problem, the present invention provides a tissue compression device, including a pump, a liquid storage chamber, catheters, and a compression device, where the liquid storage chamber is coupled to the pump, the liquid storage chamber stores fluid of a preset volume, the liquid storage chamber communicates with the compression device via the catheters, the pump provides power for fluid delivery between the liquid storage chamber and the compression device, and the compression device is used to nonlinearly compress a tissue wall relative to an axis of a tissue.

Optionally, in the embodiment of the present invention, the tissue compression device further includes a control device, where the control device is used to control working of the pump.

Optionally, in the embodiment of the present invention, a patient controls the control device in vitro.

Optionally, in the embodiment of the present invention, the compression device further includes at least one hollow flexible substrate, compression elements are disposed on the substrate, and the compression elements internally communicate with the substrate.

Optionally, in the embodiment of the present invention, the substrate has a closed or non-closed curved surface.

Optionally, in the embodiment of the present invention, the closed or non-closed curved surface includes a concave surface, and the concave surface fits the tissue.

Optionally, in the embodiment of the present invention, a cross section of the non-closed curved surface basically is arc-shaped.

Optionally, in the embodiment of the present invention, a fastening piece is disposed on the substrate, and is used to fasten the substrate to the peripheral of the tissue.

Optionally, in the embodiment of the present invention, the fastening piece includes a clasp, and the clasp can be coupled to the catheters.

Optionally, in the embodiment of the present invention, the fastening piece includes a clasp and a cartridge, and the clasp fits the cartridge, to fasten the substrate to the peripheral of the tissue.

Optionally, in the embodiment of the present invention, when the fluid in the liquid storage chamber is introduced to the compression device, the compression device nonlinearly compresses the tissue wall relative to the axis of the tissue, and when the fluid flows back to the liquid storage chamber, the compression device does not compress the tissue wall.

Optionally, in the embodiment of the present invention, the compression device includes at least three hollow compression elements, and the at least three compression elements are nonlinearly arranged along an axial direction of the tissue.

Optionally, in the embodiment of the present invention, a connection line of center points of any two compression elements is not parallel with the axis of the tissue.

Optionally, in the embodiment of the present invention, cross sections whereon the center points of any two compression elements are perpendicular to the axis of the tissue are not on a same plane.

Optionally, in the embodiment of the present invention, the compression elements each have an arc-shaped contour, and the arc-shaped contour spirally contracts towards the axis of the tissue.

Optionally, in the embodiment of the present invention, there are two catheters, one end of each catheter is coupled to a substrate, and the other end is coupled to the pump.

Optionally, in the embodiment of the present invention, a valve is disposed on each of the catheters, and the valve is used to change a flow direction of the fluid.

Optionally, in the embodiment of the present invention, the valve is a one-way valve, to control the fluid to flow in one way.

Optionally, in the embodiment of the present invention, a free end is disposed on each catheter, and the free end of the catheter can be integrated with or separated from at least one of the tissue compression device and the pump.

Optionally, in the embodiment of the present invention, a first coupling piece is disposed on the free end of the catheter, a second coupling piece is disposed on the pump, and the first coupling piece is in interference fit with the second coupling piece.

Optionally, in the embodiment of the present invention, the first coupling piece is a mounting ratchet, and the second coupling piece is a mounting hole.

Optionally, in the embodiment of the present invention, the tissue is selected from any one of a group including a penis, a urethra, a bladder, a blood vessel, a stomach, an intestine, a fallopian tube, a gall bladder, a hepatic duct, and a bile duct.

Optionally, in the embodiment of the present invention, the tissue compression device further includes a power supply, where the power supply is coupled to the pump, and supplies electric energy to the pump.

Optionally, in the embodiment of the present invention, the tissue compression device further includes a control device, where the control device is used to control working of the pump.

Optionally, in the embodiment of the present invention, the control device is a limit switch, and the limit switch is connected in series to the pump, and is used to limit stroke of the pump.

Optionally, in the embodiment of the present invention, a patient controls the control device in vitro.

Optionally, in the embodiment of the present invention, the control device includes an in vitro remote control device and an in vivo sensing device, and the in vivo sensing device is used to implement wireless coupling to the in vitro wireless remote control device, and control the compression device based on a coupling signal.

Optionally, in the embodiment of the present invention, the power supply is coupled to the in vivo sensing device, and the in vitro remote control device and the in vivo sensing device charge the power supply through wireless coupling.

Optionally, in the embodiment of the present invention, the tissue compression device further includes a sensor, where the sensor is used to sense a physiological parameter of the inside of the patient's body and/or a functional parameter of the tissue compression device, and send the physiological parameter and/or the functional parameter to the control device, and the control device adjusts delivery of the fluid based on the physiological parameter and/or the functional parameter.

Optionally, in the embodiment of the present invention, the control device is an in vitro wireless remote control device.

Optionally, in the embodiment of the present invention, the physiological parameter is pressure inside the patient's body, and the functional parameter is pressure of the compression device.

Optionally, in the embodiment of the present invention, the pump delivers, driven by the electric energy, the fluid in the liquid storage chamber to the compression device via a catheter, and delivers, driven by mechanical energy, the fluid back to the liquid storage chamber via a catheter.

Optionally, in the embodiment of the present invention, the pump includes a pump body, a pump head, and a spring, the pump head is connected to the pump body via the spring, the pump head adjusts a volume of the liquid storage chamber through reciprocating movement, and the spring at least partially drives the pump head to adjust the volume of the liquid storage chamber.

Optionally, in the embodiment of the present invention, the pump head includes a piston, the spring is coupled to the piston, the piston is coupled to an inner wall of the liquid storage chamber, and at least one of the piston and the spring delivers, driven by non-electric energy, the fluid back to the liquid storage chamber.

Optionally, in the embodiment of the present invention, the compression device further includes at least one hollow flexible substrate, compression elements are disposed on the substrate, and the compression elements internally communicate with the substrate.

Optionally, in the embodiment of the present invention, the substrate has a closed or non-closed curved surface.

Optionally, in the embodiment of the present invention, the closed or non-closed curved surface includes a concave surface, and the concave surface fits a tissue.

Optionally, in the embodiment of the present invention, a cross section of the non-closed curved surface basically is arc-shaped.

Optionally, in the embodiment of the present invention, a fastening piece is disposed on the substrate, and is used to fasten the substrate to the peripheral of the tissue.

Optionally, in the embodiment of the present invention, the fastening piece includes a clasp, and the clasp can be coupled to the catheter.

Optionally, in the embodiment of the present invention, the fastening piece includes a clasp and a cartridge, and the clasp fits the cartridge, to fasten the substrate to the peripheral of the tissue.

Optionally, in the embodiment of the present invention, when the fluid in the liquid storage chamber is introduced to the compression device, the compression device nonlinearly compresses the tissue wall relative to the axis of the tissue, and when the fluid flows back to the liquid storage chamber, the compression device does not compress the tissue wall.

Optionally, in the embodiment of the present invention, the compression device includes at least three hollow compression elements, and the at least three compression elements are nonlinearly arranged along an axial direction of the tissue.

Optionally, in the embodiment of the present invention, a connection line of center points of any two compression elements is not parallel with the axis of the tissue.

Optionally, in the embodiment of the present invention, cross sections whereon the center points of any two compression elements are perpendicular to the axis of the tissue are not on a same plane.

Optionally, in the embodiment of the present invention, the compression elements each have an arc-shaped contour, and the arc-shaped contour spirally contracts towards the axis of the tissue.

Optionally, in the embodiment of the present invention, there are two catheters, one end of each catheter is coupled to a substrate, and the other end is coupled to the pump.

Optionally, in the embodiment of the present invention, a valve is disposed on each of the catheters, and the valve is used to change a flow direction of the fluid.

Optionally, in the embodiment of the present invention, the valve is a one-way valve, to control the fluid to flow in one way.

Optionally, in the embodiment of the present invention, the power supply supplies electric energy to the valve, and the control device controls the valve to change a flow direction of the fluid.

Optionally, in the embodiment of the present invention, a free end is disposed on each catheter, and the free end of the catheter can be integrated with or separated from at least one of the tissue compression device and the pump.

Optionally, in the embodiment of the present invention, a first coupling piece is disposed on the free end of the catheter, a second coupling piece is disposed on the pump, and the first coupling piece is in interference fit with the second coupling piece.

Optionally, in the embodiment of the present invention, the first coupling piece is a mounting ratchet, and the second coupling piece is a mounting hole.

Optionally, in the embodiment of the present invention, the tissue compression device further includes a control device, where the control device is used to control working of the pump.

Optionally, in the embodiment of the present invention, the control device is a limit switch, and the limit switch is connected in series to the pump, and is used to limit stroke of the pump.

Optionally, in the embodiment of the present invention, a patient controls the control device in vitro.

Optionally, in the embodiment of the present invention, the control device includes an in vitro remote control device and an in vivo sensing device, and the in vivo sensing device is used to implement wireless coupling to the in vitro wireless remote control device, and control the compression device based on a coupling signal.

Optionally, in the embodiment of the present invention, the power supply is coupled to the in vivo sensing device, and the in vitro remote control device and the in vivo sensing device charge the power supply through wireless coupling.

Optionally, in the embodiment of the present invention, the tissue compression device further includes a sensor, where the sensor is used to sense a physiological parameter of the inside of the patient's body and/or a functional parameter of the tissue compression device, and send the physiological parameter and/or the functional parameter to the control device, and the control device adjusts delivery of the fluid based on the physiological parameter and/or the functional parameter.

Optionally, in the embodiment of the present invention, the control device is an in vitro wireless remote control device.

Optionally, in the embodiment of the present invention, the physiological parameter is pressure inside the patient's body, and the functional parameter is pressure of the compression device.

Optionally, in the embodiment of the present invention, the tissue is selected from any one of a group including a penis, a urethra, a bladder, a blood vessel, a stomach, an intestine, a fallopian tube, a gall bladder, a hepatic duct, and a bile duct.

In the present invention, the tissue compression device has a simple structure, occupies small space, is easy to be implanted, and has low power consumption and a simple operation.

In the present invention, the compression device of the tissue compression device nonlinearly compresses the tissue wall relative to the axis of the tissue. For example, at least three hollow compression elements are disposed on the compression device, and the at least three compression elements are nonlinearly arranged along the axial direction of the tissue. It is a virtue of such arrangement that can avoid 360° circumferentially compressing exerted by a tissue constraint mechanism in the prior art to a tissue, therefore, ischemic necrosis will very easily occur on the tissue and bring serious complication.

The connection line of the center points of any two compression elements is not parallel with the axis of the tissue, to avoid repeatedly compress of the two compression elements to a same part of the tissue at the same time.

The compression elements do not act on a same cross section that is perpendicular to the axis of the tissue. In other words, a cross section whereon at least one compression element is perpendicular to the axis and is not on a same plane with the cross sections whereon the other compression elements are perpendicular to the axis, therefore, pressure exerted on the tissue is further dispersed, and pressure from different directions is prevented from acting on a same segment of the tissue.

The compression elements are disposed on the substrate that basically has the arc-shaped contour. The compression elements are distributed on the substrate, so that the arc-shaped substrate can stably surround the tissue. The substrate bent to the arc shape does not directly compress the tissue, and the compression elements fastened to the substrate compress the tissue only when expanding under pressure, to control a flow of urine in the tissue, and avoid ischemic necrosis of the tissue.

The power supply, the pump, and the liquid storage chamber are of an integral structure, and the pump head is connected to the pump body via the spring. The pump head adjusts the volume of the liquid storage chamber through reciprocating movement. The spring at least partially drives the pump head to adjust the volume of the liquid storage chamber. The pump 301 pumps water out when driven by a motor, and pumps water back under tensile force of the spring, or under the action of a piston, or under the cooperated action of the spring and the piston, to reduce power consumption to some extent, and improve efficiency.

Preferably, the tissue compression device further includes the sensor, where the sensor is used to sense the physiological parameter of the inside of the patient's body and/or the functional parameter of the tissue compression device, and send the physiological parameter and/or the functional parameter to the control device, and the control device adjusts delivery of the fluid based on the physiological parameter and/or the functional parameter. The tissue compression device is in a powered-on and standby state. When the physiological parameter of the inside of the patient's body and/or the functional parameter of the tissue compression device exceeds a threshold or starts to increase, for example, the patient coughs or sneezes, the sensor sends a signal to the controller, and the compression device rapidly compresses the tissue, to avoid a phenomenon of liquid leakage of the tissue when the patient coughs or sneezes.

### BRIEF DESCRIPTION OF THE DRAWINGS

To describe the technical solutions in the embodiments of the present invention or in the prior art more clearly, the following briefly describes the accompanying drawings required for describing the embodiments or the prior art. Apparently, the accompanying drawings in the following description show merely some embodiments recorded in the embodiments of the present invention, and persons of ordinary skill in the art may derive other drawings from these accompanying drawings without creative efforts.
Figure 1 is a schematic diagram of an embodiment of a tissue compression device according to the present invention.
Figure 2A to Figure 2D are schematic diagrams of four embodiments of a compression device in Figure 1.
Figure 3 is a schematic diagram of a preferred embodiment based on Figure 2A to Figure 2D.
Figure 4 is a schematic diagram of Embodiment 1 of a tissue compression device corresponding to Figure 3.
Figure 5 is a schematic diagram when a tissue compression device corresponding to Figure 2B is in an unused state.
Figure 6 is a schematic diagram when a tissue compression device corresponding to Figure 2B is in a used state.
Figure 7 is a schematic diagram of Embodiment 2 of a tissue compression device corresponding to Figure 3.
Figure 8 is a schematic diagram after a substrate of a tissue compression device corresponding to Figure 7 is bent.
Figure 9 is a schematic diagram of a compression device in Figure 7.
Figure 10 is a schematic diagram when a tissue compression device corresponding to Figure 7 is in an unused state.
Figure 11 is a schematic diagram when a tissue compression device corresponding to Figure 7 is in a used state.
Figure 12 is a schematic structural diagram of a catheter in Figure 1.
Figure 13 is a schematic amplified structural diagram of a catheter in Figure 12.
Figure 14 is a schematic structural diagram of an embodiment of a pump in Figure 1.
Figure 15 is a schematic diagram of a preferred embodiment of a tissue compression device according to the present invention.

### DETAILED DESCRIPTION

Certainly, implementing any technical solution of the embodiments of the present invention does not need to achieve all the foregoing advantages at the same time.

To make persons skilled in the art to better understand the technical solutions of the embodiments of the present invention, the following clearly and completely describes the technical solutions of the embodiments of the present invention with reference to the accompanying drawings in the embodiments of the present invention. Apparently, the described embodiments are merely some rather than all embodiments of the present invention. All other embodiments obtained by persons of ordinary skill in the art based on the embodiments of the present invention shall belong to the protection scope of the embodiments of the present invention.

The "axial direction" is a direction that extends along a length direction of a tissue. The "fluid" includes, but not limited to, liquid and gas, for example, sterile water, normal saline, and air.

The following further describes a specific implementation of the embodiments of the present invention with reference to the accompanying drawings of the embodiments of the present invention.

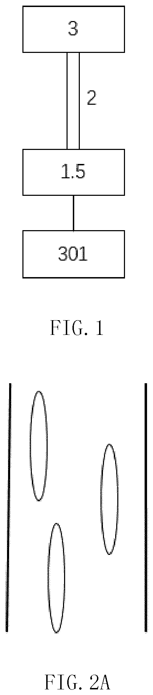

Referring to Figure 1, in a specific implementation of the present invention, the tissue compression device includes a pump 301, a liquid storage chamber 1.5, catheters 2, and a compression device 3. The liquid storage chamber 1.5 is coupled to the pump 301, the liquid storage chamber 1.5 stores fluid of a preset volume, the liquid storage chamber 1.5 communicates with the compression device 3 via the catheters 2, the pump 301 provides power for liquid delivery between the liquid storage chamber 1.5 and the compression device 3, and the compression device 3 is used to nonlinearly compress a tissue wall relative to an axis of a tissue.

Optionally, in the embodiment of the present invention, when the fluid in the liquid storage chamber is introduced to the compression device 3, the compression device 3 nonlinearly compresses the tissue wall relative to the axis of the tissue, and when the fluid flows back to the liquid storage chamber 1.5, the compression device 3 does not compress the tissue wall.

Optionally, in the embodiment of the present invention, the compression device 3 includes at least three hollow compression elements, and the at least three compression elements are nonlinearly arranged along an axial direction of the tissue.

That the at least three compression elements are nonlinearly arranged along the axial direction of the tissue includes, but not limited to, that a connection line of center points of the at least three compression elements is not parallel with the axis of the tissue. For example, after the tissue stretches, the axis is a straight line, and the connection line of the center points of the at least three compression elements is a straight line or a curve, and intersects with the axis of the tissue, or an extended line of the connection line intersects with the axis of the tissue.

Optionally, in an embodiment of the present invention, a connection line of center points of any two compression elements is not parallel with the axis of the tissue.

Optionally, in an embodiment of the present invention, cross sections whereon the center points of any two compression elements are perpendicular to the axis of the tissue are not on a same plane. Specifically, planes whereon the center points of any two compression elements are perpendicular to the axis of the tissue are not on a same plane.

Preferably, the compression elements do not act on a same cross section that is perpendicular to the axis of the tissue. In other words, a cross section whereon at least one compression element is perpendicular to the axis and is not on a same plane with the cross sections whereon the other compression elements are perpendicular to the axis. It is a virtue of such arrangement that can avoid 360° circumferentially compressing exerted by a tissue constraint mechanism in the prior art to a tissue, therefore, ischemic necrosis will very easily occur on the tissue and bring serious complication.

Optionally, in an embodiment of the present invention, the compression device 3 further includes at least one hollow flexible substrate, compression elements are disposed on the substrate, and the compression elements internally communicate with the substrate.

Optionally, in the embodiment of the present invention, the substrate has a closed or non-closed curved surface.

Optionally, in the embodiment of the present invention, the closed or non-closed curved surface includes a concave surface, and the concave surface fits the tissue.

Optionally, in the embodiment of the present invention, a cross section of the non-closed curved surface basically is arc-shaped.

Optionally, in the embodiment of the present invention, a fastening piece is disposed on the substrate, and is used to fasten the substrate to the peripheral of the tissue.

Preferably, the fastening piece includes a clasp, and the clasp can be coupled to the catheter.

Preferably, the fastening piece includes a clasp and a cartridge, and the clasp fits the cartridge, to fasten the substrate to the peripheral of the tissue.

Optionally, in the embodiment of the present invention, the compression elements each have an arc-shaped contour, and the arc-shaped contour spirally contracts towards the axis of the tissue.

Optionally, in the embodiment of the present invention, the tissue compression device further includes a control device, where the control device is used to control working of the pump.

Optionally, in the embodiment of the present invention, the control device is a limit switch, and the limit switch is connected in series to the pump, and is used to limit stroke of the pump. For example, the limit switch usually is in a normally closed state. When the pump completely pumps the fluid into the compression device, the limit switch is disconnected, and the pump stops working.

Optionally, in the embodiment of the present invention, there are two catheters, one end of each catheter is coupled to a substrate, and the other end is coupled to the pump.

Optionally, in the embodiment of the present invention, a valve is disposed on each of the catheters, and the valve is used to change a flow direction of the fluid.

Preferably, the power supply supplies electric energy to the valve, and the control device controls the valve to change a flow direction of the fluid. In a preferred embodiment of the present invention, the valve is an electromagnetic valve. The valve may be controlled by the control device. For example, when the pump pumps the fluid from the liquid storage chamber to the compression device, the valve can be opened automatically under an impact effect of the fluid without being driven by electric energy. When the fluid flows back to the liquid storage chamber from the compression device, the valve is powered on, and the control device controls the valve to be opened, to complete discharging of the compression device.

Preferably, a one-way valve is disposed on each catheter, to control the fluid to flow in one way.

Optionally, in the embodiment of the present invention, a free end is disposed on each catheter, and the free end of the catheter can be integrated with or separated from at least one of the tissue compression device and the pump.

Optionally, in the embodiment of the present invention, a first coupling piece is disposed on the free end of the catheter, a second coupling piece is disposed on the pump, and the first coupling piece is in interference fit with the second coupling piece.

Preferably, the first coupling piece is a mounting ratchet, and the second coupling piece is a mounting hole.

Optionally, in the embodiment of the present invention, the tissue is selected from any one of a group including a penis, a urethra, a bladder, a blood vessel, a stomach, an intestine, a fallopian tube, a gall bladder, a hepatic duct, and a bile duct.

In an embodiment of the present invention, the pump 301 is a manual pump. A patient squeezes the pump 301 with a hand, so that the pump 301 pushes the fluid in the liquid storage chamber 1.5 to flow into cavities of the compression elements 3.1 via a catheter 2, so that the compression elements 3.1 expand, to compress the tissue. In addition, the fluid may be manually controlled to flow back to the liquid storage chamber 1.5 from the cavities of the compression elements 3.1. Optionally, in an embodiment of the present invention, a one-way valve is disposed on each catheter, to control the fluid to flow in one way.

The catheter 2 is a hollow catheter, and a wall lining may have a strength enhancing structure, for example, a mesh structure that enhances strength. There are one or more catheters 2. Preferably, there are two catheters 2, one end of each catheter is coupled to the substrate 3a, and the other end is coupled to the pump 301.

In an embodiment of the present invention, the pump 301 is an electric pump. Optionally, in the embodiment of this application, the tissue compression device further includes a power supply, where the power supply is coupled to the pump, and supplies electric energy to the pump.

Optionally, in the embodiment of the present invention, the pump delivers, driven by the electric energy, the fluid in the liquid storage chamber to the compression device via a catheter, and delivers, driven by mechanical energy, the fluid back to the liquid storage chamber via a catheter.

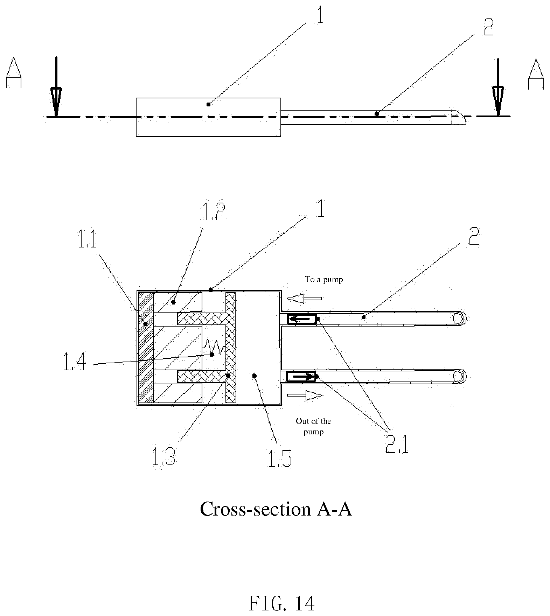

As shown in Figure 14, optionally, in the embodiment of the present invention, the pump includes a pump body 1.2, a pump head 1.3, and a spring 1.4, the pump head 1.3 is connected to the pump body 1.2 via the spring 1.4, the pump head 1.3 adjusts a volume of the liquid storage chamber through reciprocating movement, and the spring 1.4 at least partially drives the pump head 1.3 to adjust the volume of the liquid storage chamber.

Optionally, in the embodiment of the present invention, the pump head 1.3 includes a piston, the spring 1.4 is coupled to the piston, the piston is coupled to an inner wall of the liquid storage chamber, and at least one of the piston and the spring 1.4 delivers, driven by non-electric energy, the fluid back to the liquid storage chamber.

The liquid storage chamber 1.5 and the pump 301 may be two separate devices or integrated as an integral structure. The pump 301 may be a one-way pump 301 or a bidirectional pump 301.

The following embodiment describes a case in which the liquid storage chamber 1.5 and the pump 301 are two separate devices.

The pump 301 is located between the liquid storage chamber 1.5 and the compression device 3, the pump 301 is connected to each of the liquid storage chamber 1.5 and the compression device 3 via a catheter, and the pump 301 may be a one-way pump 301 or a bidirectional pump 301. The pump 301 pumps the fluid out of the liquid storage chamber 1.5, and delivers the fluid to the compression elements on the compression device, so that the compression elements expand. When the tissue needs to restore to a smooth state, the pump 301 pumps the fluid out of the compression elements, and delivers the fluid back to the liquid storage chamber 1.5. One or more valves may be disposed between the pump 301 and the liquid storage chamber 1.5, or between the pump 301 and the tissue, to control flowing of the fluid. Optionally, in the embodiment of the present invention, the pump 301 is connected to the liquid storage chamber 1.5 via two catheters, one end of each catheter is coupled to the substrate, and the other end is coupled to the pump.

Optionally, in the embodiment of the present invention, a one-way valve is disposed on each catheter, to control the fluid to flow in one way.

The following embodiment describes a case in which the liquid storage chamber 1.5 and the pump 301 are of an integral structure.

The pump 301 and the liquid storage chamber 1.5 are integrated together, and a membrane, a piston, or another appropriate structure is disposed between the pump and the liquid storage chamber to separate the pump and the liquid storage chamber. When the pump 301 is started, the pump 301 squeezes the liquid storage chamber 1.5, so that a volume of the liquid storage chamber 1.5 is decreased, and the fluid flows out of the liquid storage chamber 1.5.

The liquid storage chamber 1.5 and the pump 301 are separately (separate devices) or jointly (the integral structure) covered by a housing. The housing is made of a material having biological compatibility, for example, stainless steel, titanium alloy, cobalt-chromium alloy, or silica gel. A material such as poly-p-xylylene, Teflon, polysaccharide polymer, or modified silicone may be sprayed or coated on a surface of the housing, to improve performance of the surface of the housing, and enhance biological compatibility and stability.

Optionally, in an embodiment of the present invention, the tissue compression device further includes a power supply, where the power supply is coupled to the pump 301, and supplies electric energy to the pump 301. The power supply is a battery or another device that can supply electric energy.

The power supply may be separated from the liquid storage chamber 1.5 and the pump 301, or may be integrated with the pump 301, or may be integrated with the liquid storage chamber 1.5 and the pump 301. The power supply, the liquid storage chamber 1.5, and the pump 301 may be disposed in a scrotum or an inferior belly.

Preferably, the tissue compression device further includes a control device, and the control device is used to control working of the pump 301. Optionally, in the embodiment of the present invention, a patient controls the control device in vitro. In the embodiment, the control device may be a switch.

In an embodiment of the present invention, the control device is disposed in the scrotum. When the patient exerts force on the scrotum in vitro and starts the switch, the power supply supplies power such as electric energy to the pump 301. The pump 301 is started, and pushes the fluid in the liquid storage chamber 1.5 to flow into the cavities of the compression elements 3.1 via the catheter 2. When the switch is disconnected, the fluid flows back to the liquid storage chamber 1.5 from the cavities of the compression elements 3.1.

Preferably, the pump 301 further includes a motor that can convert electric energy into mechanical movement or a dynamical system that includes a motor and a transmission mechanism. The motor may be a rotating motor or a linear motor, and the transmission mechanism may be a common transmission mechanism such as a gear, a turbine, or a ball screw.

Optionally, in an embodiment of the present invention, the pump 301, driven by the electric energy, delivers the fluid in the liquid storage chamber 1.5 to the compression device 3 via the catheter, and delivers, driven by the mechanical energy, the fluid back to the liquid storage chamber 1.5 via the catheter 2.

Optionally, in an embodiment of the present invention, the control device includes an in vitro remote control device and an in vivo sensing device, and the in vivo sensing device is used to implement wireless coupling to the in vitro wireless remote control device, and control the compression device based on a coupling signal.

Optionally, in the embodiment of the present invention, the power supply is coupled to the in vivo sensing device, and the in vitro remote control device and the in vivo sensing device charge the power supply through wireless coupling.

Optionally, in the embodiment of the present invention, the tissue compression device further includes a sensor, where the sensor is used to sense a physiological parameter of the inside of the patient's body and/or a functional parameter of the tissue compression device, and send the physiological parameter and/or the functional parameter to the control device, and the control device adjusts delivery of the fluid based on the physiological parameter and/or the functional parameter. Preferably, the control device is an in vitro wireless remote control device. Preferably, the physiological parameter is pressure inside the patient's body, and the functional parameter is pressure of the compression device.

The liquid storage chamber 1.5 and the pump 301 are separately (separate devices) or jointly (the integral structure) covered by the housing. The housing is made of the material having biological compatibility, for example, stainless steel, titanium alloy, cobalt-chromium alloy, or silica gel. The material such as poly-p-xylylene, Teflon, polysaccharide polymer, or modified silicone may be sprayed or coated on the surface of the housing, to improve performance of the surface of the housing, and enhance biological compatibility and stability.

The compression elements may be disposed on a coating layer or the hollow substrate.

When the compression elements are disposed on the coating layer, the compression elements surround the peripheral of the tissue, and the compression elements and the coating layer of an outer surface have biological compatibility and extension performance, so that the compression elements have good compatibility and stability with the surrounding tissue. The coating layer is made of a high-molecular polymer material, for example, PTFE, TPU, PU, silica gel, silicone, a thermoplastic elastomer, a modified material, or a composite material.

Figure 2A to Figure 2D are schematic diagrams of four embodiments of a compression device in Figure 1.

Referring to Figure 2A, the compression device 3 includes three hollow compression elements, each compression element is a bump that extends circumferentially around the tissue, and the cross section whereon the bump passes through a center point of a compression element is shaped in, but not limited to, a semi-circle, a trapezoid, a triangle, a circle, a quadrangle, an arc, or a quasi-oval.

The compression elements may directly communicate with the pump 301, or may communicate with the pump 301 via a common transit mechanism. The pump 301 may directly deliver the fluid to the compression elements via the catheter 2, so that the compression elements expand to compress the tissue 5, to control a flow of fluid in the tissue. Alternatively, the pump 301 may first deliver the fluid to a transit mechanism, and then the transmit mechanism allocates the fluid to the compression elements. The transit mechanism may be any appropriate container, and the transit mechanism may be located between the pump 301 and the compression elements, or may be connected to one of the pump and the compression elements.

Cross sections whereon the center points of any two compression elements are perpendicular to the axis of the tissue are not on a same plane. Therefore, the cross section of any segment of the tissue will not show entire cross sections (not shown) whereon two or more of the compression elements passes through center points thereof at the same time. Such design effectively ensures that any two compression elements will not repeatedly compress the tissue in the axial direction.

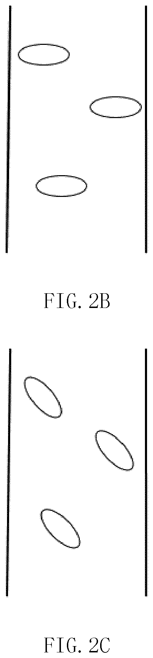

Referring to Figure 2B, the compression device 3 includes three hollow compression elements, and the compression elements each have a contour that extends horizontally. In other words, the compression elements each extends horizontally along a direction perpendicular to the axis of the tissue. The contour may be of an oval shape, an irregular shape, a cylindrical shape, a trapezoid shape, a conical shape, or the like.

Referring to Figure 2c, the compression device 3 includes three hollow compression elements, and the compression elements each have a contour that extends obliquely. In other words, the compression elements each extend along a direction that is not parallel with or perpendicular to the axis of the tissue. The contour may be of an oval shape, an irregular shape, a cylindrical shape, a trapezoid shape, a conical shape, or the like.

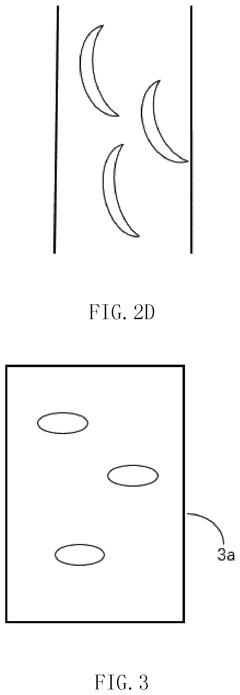

Referring to Figure 2D, the compression device 3 includes three hollow compression elements, the compression elements each have an arc-shaped contour, and the arc-shaped contour spirally contracts towards the tissue. Different from Figure 2A to Figure 2C, an axis of the arc-shaped contour is not a straight line.

Figure 3 is a schematic diagram of a preferred embodiment based on Figure 2A to Figure 2D. The compression device 3 further includes at least one hollow flexible substrate 3a, the compression elements are disposed on the substrate 3a, and the compression elements internally communicate with the substrate 3a. The pump 301 may deliver the fluid to the substrate 3a via the catheter 2, and when pressure of the fluid reaches a particular threshold, the fluid in the substrate 3a enables the compression elements to expand, to compress the tissue 5, to control a flow of the fluid in the tissue. The threshold may be pressure exerted on the substrate 3a when the substrate 3a is filled with the fluid, or may be another pressure value.

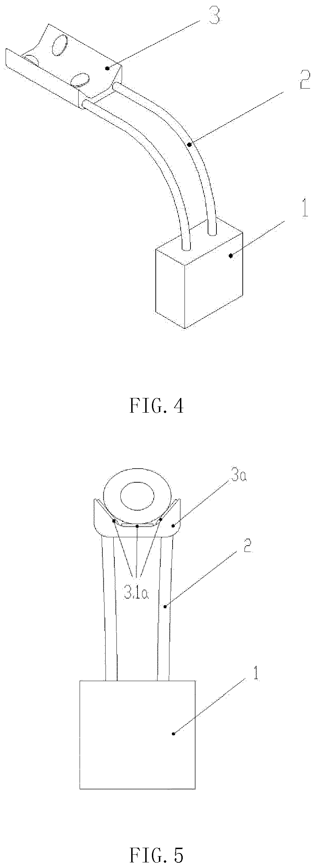

Figure 4 is a schematic diagram of embodiment 1 of a tissue compression device corresponding to Figure 3. The compression device 3 includes a substrate and compression elements located on the substrate. The substrate has an non-closed curved surface. In the embodiment, the non-closed curved surface includes a concave surface, and the concave surface fits the tissue. The substrate is a sealed hollow structure whose entire cross section is arc-shaped. In an embodiment of the present invention, the substrate has a contour that is basically arc-shaped. An internal cavity of the substrate communicates with the catheter 2, and the compression elements are distributed on and fastened to the substrate.

A shape of an outer surface of the substrate is not limited to a combination of a concave shape and a horizontal surface, and may further include one or more of a convex shape, a plane, a curved surface, a mesh plane or a curved surface, an irregular surface or any other appropriate shape, or a combination thereof. Preferably, the outer surface of the substrate has a concave upper surface and a horizontal lower surface, and the upper surface and the lower surface are connected via vertical planes. Two catheters connecting to the substrate are separately located on two right-angle regions of the lower surface, to ensure that thickness between the upper surface and the lower surface is minimized.

Optionally, in the embodiment of this application, a fastening piece is disposed on the substrate, and is used to fasten the substrate to the peripheral of the tissue. For example, the substrate may include two hollow substrates that can be bent, and the compression elements are disposed on at least one substrate. A cartridge is fastened to each end of one substrate, a clasp is fastened to each end of the other substrate, and each clasp can be fastened to a neighboring cartridge, to fasten two substrates to the peripheral of the tissue through cooperation between the clasp and the cartridge, and avoid that the substrate completely covers the tissue for a long time, and causes tissue damages. Preferably, a diameter of the substrate is slightly greater than a diameter of the tissue, so that the substrate may slide relative to the tissue, to further avoid that the substrate repeatedly compresses a same segment of the tissue for a long time. The clasp and the cartridge may be of any design or shape. In an embodiment of the present invention, one end of each clasp is fastened to the substrate, the other end is an annular loop, a corresponding groove is formed on the cartridge, and the annular loop can be exactly interlinked with the groove. In another embodiment of the present invention, a material that can be pasted or attracted is disposed on the cartridge, one end of the clasp is fastened to the substrate, and the other end can be pasted to or attracted by the material that can be pasted or attracted. The device 1 includes the liquid storage chamber and the pump.

Figure 5 is a schematic diagram when a tissue compression device corresponding to Figure 2B is in an unused state. When the tissue compression device is in the unused state, the tissue is in an open state, and the substrate 3a and the compression elements 3.1a are in a fatigued and weak state.

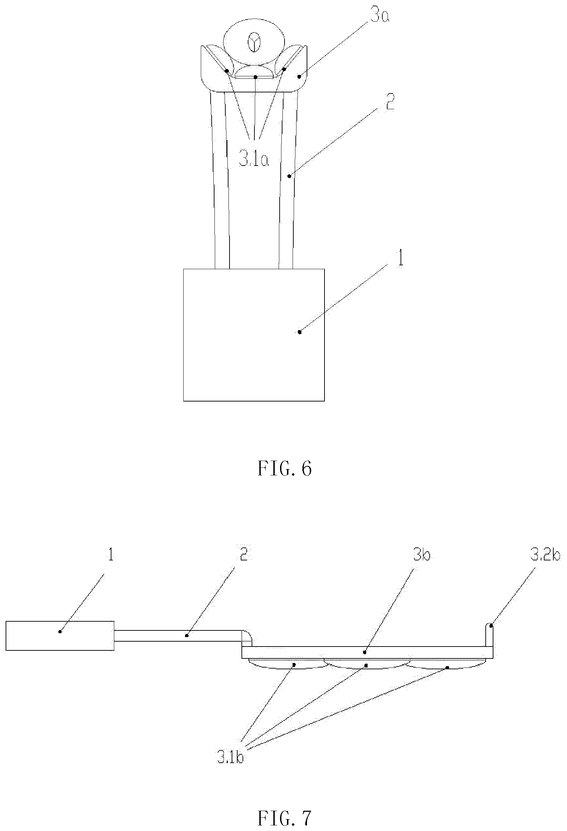

Figure 6 is a schematic diagram when a tissue compression device corresponding to Figure 2B is in a used state. After the tissue compression device is started, fluid pumped out by the pump 301 may be delivered to the substrate 3a via the catheter 2, and when pressure of the fluid reaches a particular threshold, the fluid in the substrate 3a enables the compression elements 3.1a to expand, to compress the tissue, to control a flow of the fluid in the tissue.

Figure 7 is a schematic diagram of Embodiment 2 of a tissue compression device corresponding to Figure 3. Different from embodiment 1 corresponding to Figure 4, the substrate 3b has a closed curved surface. A fastening piece is disposed on the substrate 3b, and is used to fasten the substrate 3b to the peripheral of the tissue. In the embodiment, the fastening piece is a clasp 3.2b, and the clasp 3.2b can be coupled to the catheter 2, to bend the substrate 3b and fasten the substrate 3b to the peripheral of the tissue. The clasp 3.2b and the cartridge (located on the catheter 2) may be of any design or shape. In an embodiment of the present invention, one end of the clasp 3.2b is fastened to the substrate, the other end is an annular loop, a corresponding groove is formed on the cartridge, and the annular loop can be exactly interlinked with the groove. In another embodiment of the present invention, a material that can be pasted or attracted is disposed on the cartridge, one end of the clasp 3.2b is fastened to the substrate, and the other end can be pasted to or attracted by the material that can be pasted or attracted.

Preferably, one end of the catheter 2 is a fastening bayonet, and one end is a free end 2.2. The fastening bayonet of the catheter 2 is fastened to the substrate, and the free end 2.2 of the catheter 2 may pass through the fastening bayonet of the catheter 2, and is firmly fastened relative to the bayonet, so that the substrate that is bent to a circular ring shape can stably surround the tissue. However, the substrate that is bent to an arc shape does not directly compress the tissue, instead, the substrate compresses the tissue only when the compression elements fastened to the substrate expand under pressure, to control a flow of urine in the tissue.

The internal of the compression element is a hollow communication structure. The liquid pumped out by the pump 301 is delivered to the substrate via the catheter 2. When pressure of the liquid reaches a particular threshold, the liquid in the substrate can enable the compression elements to expand, to compress the tissue, and control a flow of urine in the tissue.

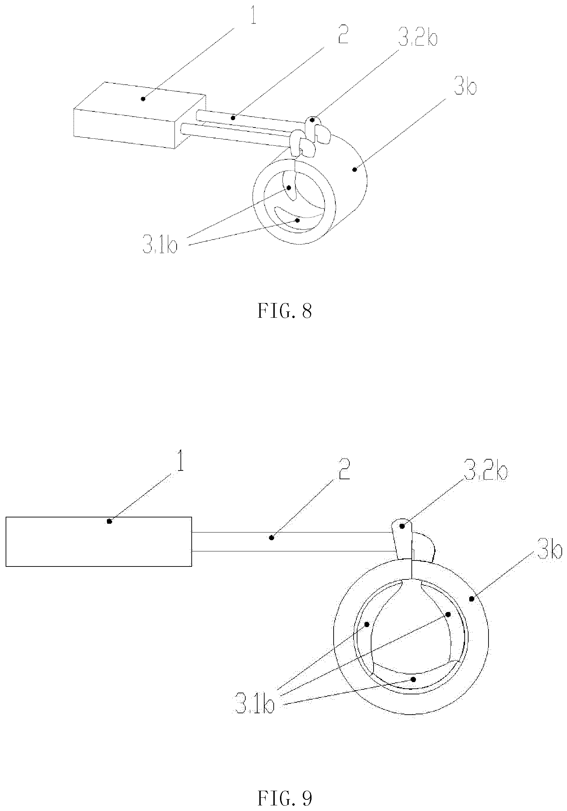

Referring to Figure 8 and Figure 9, the substrate 3b is of a planar closed hollow structure, an internal cavity of the substrate communicates with the catheter 2, and the substrate 3b is made of an elastic material. The substrate 3b can be bent to a circular ring shape based on needs, and the tissue is allowed to pass through a middle part of the substrate 3b that is bent to the circular ring shape.

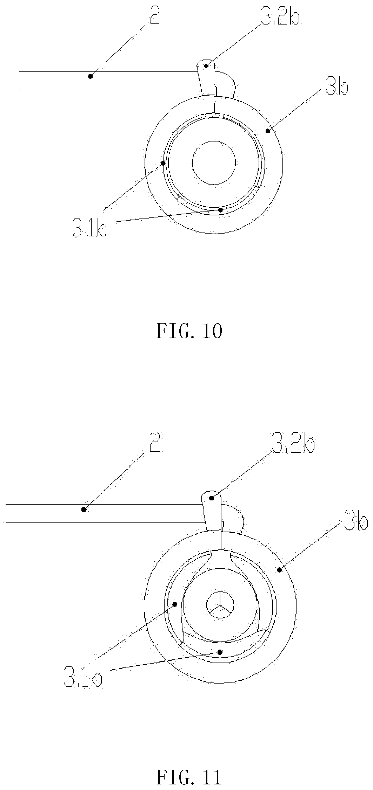

Referring to Figure 10, when the tissue compression device does not work, the compression elements 3.1b are arranged on an inner side of the circular ring-shaped substrate 3b.

Referring to Figure 11, when the compression elements 3.1b expand, the compression elements can compress the tissue that passes through the circular ring-shaped substrate 3b, to control a flow of urine in the tissue.

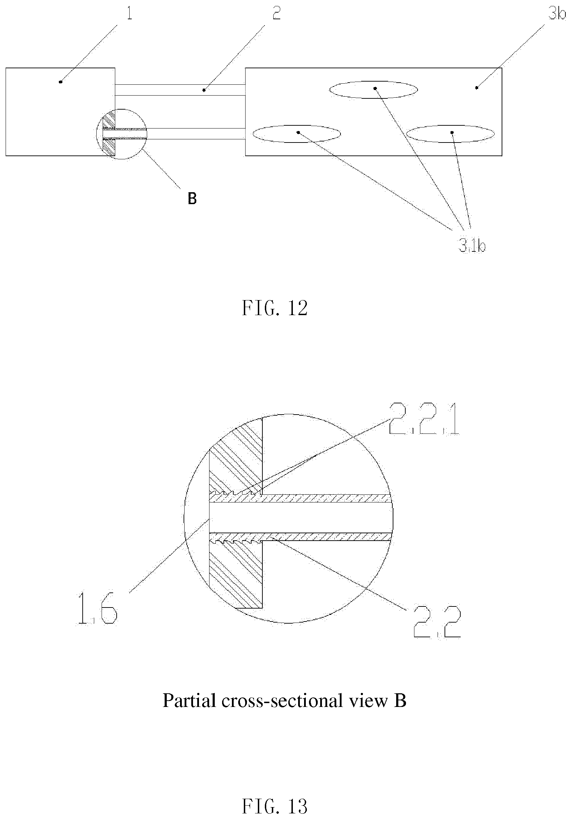

Figure 12 is a schematic structural diagram of the catheter 2 in Figure 1. Figure 13 is a schematic amplified structural diagram of the catheter 2 in Figure 12. The free end 2.2 is disposed on the catheter 2, and the free end 2.2 of the catheter 2 can be integrated with or separated from at least one of the tissue compression device and the pump 301. One end or two ends of the catheter 2 are set to the free end 2.2. For the convenience of mounting, only one end of the catheter 2 can be set to the free end 2.2. The free end 2.2 can be mounted or disassembled.

Preferably, the free end 2.2 of the catheter 2 can be mounted onto or disassembled from the pump 301, so that during the implantation, after the pump 301 and the compression elements 3.1 are separately implanted, the pump and the compression elements are connected via the free end 2.2 of the catheter 2, and an operation is simple.

Optionally, there are two catheters 2, one end of each catheter is coupled to a substrate, and the other end is coupled to the pump 301. A one-way valve is disposed on each catheter 2, to control the fluid to flow in one way.

As shown in Figure 13, a first coupling piece is disposed on the free end 2.2 of the catheter 2, a second coupling piece is disposed on the pump 301, and the first coupling piece is in interference fit with the second coupling piece. Preferably, the first coupling piece is a mounting ratchet 2.2.1, and the second coupling piece is a mounting hole 1.6. The first coupling piece and the second coupling piece may be connected in another interference fit manner. During mounting, the free end 2.2 of the catheter 2 may be inserted into the mounting hole 1.6 of the pump 301, and stability of plug-in mounting can be ensured through interference fit between the mounting ratchet 2.1.1 and the mounting hole 1.6.

Figure 14 is a schematic structural diagram of an embodiment of the pump 301 in Figure 1. The power supply is coupled to the pump 301, and supplies electric energy to the pump 301. The pump 301 includes a pump body 1.2, a pump head 1.3, and a spring 1.4. The pump head 1.3 includes a piston and a push rod. The pump head 1.3 is connected to the pump body 1.2 via the spring 1.4. The pump head 1.3 adjusts a volume of the liquid storage chamber through reciprocating movement. The spring 1.4 at least partially dives the pump head 1.3 to adjust a volume of the liquid storage chamber.

The pump 301 includes a housing, a power supply, a pump body 1.2, and a pump head 1.3, and the pump and the liquid storage chamber 1.5 form an integral structure. The housing is made of a material having biological compatibility, for example, stainless steel, titanium alloy, cobalt-chromium alloy, or silica gel. A material such as poly-p-xylylene, Teflon, polysaccharide polymer, or modified silicone may be sprayed or coated on a surface of the housing, to improve performance of the surface of the housing, and enhance biological compatibility and stability. The power supply may supply electric energy to the pump body 1.2 and a switch (not shown) of the pump 301, and the power supply may be further disposed to supply electric energy to an electromagnetic valve of the catheter 2, to control on and off of a liquid flow in the catheter 2.

The pump body 1.2 is a motor that converts electric energy into mechanical movement or a dynamical system that includes a motor and a transmission mechanism. The motor may be a rotating motor or a linear motor. The transmission mechanism may be a common transmission mechanism such as a gear, a turbine, or a ball screw. In the present invention, preferably, the linear motor is used as the dynamical system of the pump body 1.2. The pump body 1.2 is used to drive the pump head 1.3 to move away from the pump body 1.2, so that the piston on the pump head 1.3 squeezes the liquid in the liquid storage chamber 1.5, and the liquid flows into the compression elements 3.1 or the substrate 3a via the catheter 2. The pump head 1.3 includes a push rod 1 and a piston. The push rod 1 is driven by the pump body 1.2, to push the piston to exert pressure on the liquid in the liquid storage chamber, and enable the liquid to flow into the compression elements 3.1 via the catheter 2. The piston and an internal wall of the liquid storage chamber 1.5 may form a sealed cavity. Under a pushing and pulling motion of the piston, the liquid in the liquid storage chamber 1.5 may flow out of the liquid storage chamber 1.5 and flow back to the liquid storage chamber 1.5. Preferably, in the present invention, an extension spring 1.4 is disposed between the pump head 1.3 and the pump body 1.2. When the pump head 1.3 is pushed by the pump body 1.2 to a maximum degree, the tension spring 1.4 is pulled, and when the pump body 1.2 is in a unexcited state and the electromagnetic valve on the catheter 2 is in a fluid flow opened state, the extension spring 1.4 slowly pulls the pump head 1.3 to a start location, and meanwhile, enables the liquid in the compression elements 3.1 to slowly flow back to the liquid storage chamber 1.5. When the pump 301 pumps water out, the pump is driven by the motor, and when the pump 301 pumps the water back, the pump is driven under tensile force of the spring 1.4 and/or under the action of the piston. Therefore, there is no other energy consumption, to fully play a role of the power supply.

The power supply is a battery or a rechargeable battery. A type of a battery 1.1 includes a lithium ion battery, a lithium-iodine battery, a graphene battery, and the like.

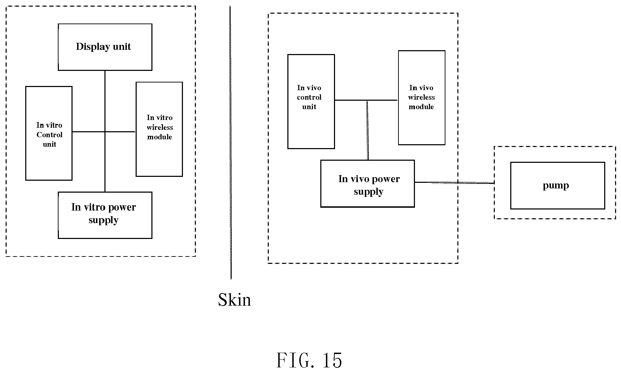

Figure 15 is a schematic diagram of embodiment 1 of a tissue compression device used to cure urinary incontinence according to the present invention. The tissue compression device includes a pump 301, a liquid storage chamber 1.5, catheters 2, and a compression device 3. The liquid storage chamber 1.5 is coupled to the pump 301, the liquid storage chamber 1.5 storage fluid of a preset volume, and the liquid storage chamber 1.5 is coupled to the compression device 3 via the catheters 2. The compression device 3 includes three hollow compression elements, and the compression elements are arranged on an arc-shaped plane of the substrate 3a in a triangular form.

The control device includes an in vitro remote control device and an in vivo sensing device, and the in vivo sensing device is used to implement wireless coupling to the in vitro wireless remote control device, and control the compression device based on a coupling signal. The power supply is coupled to the in vivo sensing device, and the in vitro remote control device and the in vivo sensing device charge the power supply through wireless coupling.

The in vitro remote control device is a remote controller, and includes a display unit, an in vitro control unit, an in vitro wireless module, and an in vitro power supply. The in vivo sensing device includes an in vivo control unit, an in vivo wireless module, and an in vivo power supply. The in vivo power supply is connected to a motor of the pump 301, to supply electric energy to the motor.

A user sends a control signal to the in vitro control unit via the display unit, the in vitro control unit sends the signal (for example, an electromagnetic wave signal, or a wireless signal of another type) to the in vitro wireless module. The in vitro wireless module is wirelessly coupled to the in vivo wireless module, and converts the wireless signal into an electric current, to charge the in vivo power supply, and the in vivo power supply supplies electric energy to the pump 301. In addition, the electric current is transmitted to the in vivo control unit, to control the pump 301 to be started.

Preferably, the tissue compression device further includes a sensor, where the sensor is used to sense a physiological parameter of the inside of the patient's body and/or a functional parameter of the tissue compression device, and send the physiological parameter and/or the functional parameter to the in vivo control device or the in vitro wireless remote control device, and the in vivo control device or the in vitro wireless remote control device adjusts delivery of the fluid based on the physiological parameter and/or the functional parameter.

Preferably, the physiological parameter is pressure inside the patient's body, and the functional parameter is pressure of the compression device.

For example, the sensor transmits the sensed pressure inside the patient's body and the sensed pressure of the compression device to the in vitro wireless remote control device in time, intermittently, or irregularly. If the pressure inside the patient's body rapidly increases or exceeds a particular threshold set by a system (for example, when the patient coughs), if the pressure of the compression device does not exceed a threshold (for example, a maximum value), the in vitro wireless remote control device automatically controls the pump to pump the fluid into the compression device, to compress the tissue, and prevent leakage of liquid in the tissue.

There are two catheters 2, which are used as pipelines to connect the pump 301 to the compression device. One-way valves having opposite liquid flowing directions are separately disposed in the two catheters 2, to ensure one way flow direction of liquid in a sealed liquid path that is constitute of the pump 301, the catheters 2, and the compression device. Therefore, an entire electric urination control device works efficiently, securely, and stably with a simple structure. One end of each catheter 2 and the compression device are of an integral structure, and the other end is a free end 2.2. A mounting ratchet 2.2.1 is disposed on the free end 2.2 of the catheter 2. During mounting, the free end 2.2 of the catheter 2 may be inserted into the mounting hole 1.6 on the pump 301. The mounting ratchet 2.2.1 is in interference fit with the mounting hole 1.6, to ensure stability of plug-in mounting.

Preferably, at least three compression elements are disposed on the substrate 3a, and the compression elements do not act on a same plane of a cross section of the tissue. In other words, at least one compression element is located on a cross section of the tissue that is different from the cross sections of the tissue whereon the other compression elements are located. It is a virtue of such arrangement that can avoid 360 ° circumferentially compressing exerted by a tissue constraint mechanism in the prior art to a tissue, therefore, ischemic necrosis will very easily occur on the tissue and bring serious complication.

When the tissue compression device of the present invention is used to cure urinary incontinence, the pump 301 is implanted inside the scrotum of the patient or subcutaneously in the inferior belly through a surgical operation. Then, an incision is made on the skin on the penis tissue of the patient, the tissue and the skin fascia are separated through blunt dissection, the compression device is placed inside, surfaces of the compression elements 3.1 fit the tissue, and then the free end 2.2 of each catheter 2 is plugged into the mounting hole 1.6 of the pump 301, to complete the mounting. The skin is seamed after it is tested through the remote control device 4 that the electric urination control device works effectively, to complete an implantation operation.

In another embodiment 2, a clasp is disposed on the substrate 3b, one end of each catheter 2 is a fastening bayonet, one end is a free end 2.2, the fastening bayonet of the catheter 2 is fastened to the substrate 3b, and the free end 2.2 of the catheter 2 may pass through the fastening bayonet of the catheter 2, and is fastened relative to the bayonet, so that the substrate 3b bent to a circular ring shape may stably surround the tissue.

The substrate 3b is of a planar sealed hollow structure, and an internal cavity communicates with the catheters 2. In the embodiment, three compression elements 3.1b are arranged on one surface of the substrate 3b, and the compression elements 3.1b are nonlinearly arranged along the tissue. In other words, a connection line of center points of any two compression elements 3.1b is not parallel with the axis of the tissue.

During use, the electric urination control device is taken, and the pump 301 is implanted inside the scrotum of the patient or subcutaneously in the inferior belly through a surgical operation. Then, an incision is made on the skin on the penis tissue, the tissue and the neighboring tissue are separated through blunt dissection, a length of the tissue is equivalent to a width of the substrate 3b, and is approximately 10 mm, and surfaces of the compression elements 3.1 on the substrate 3a directly face the tissue. The substrate 3b is bent to a circular ring shape around the tissue, and surrounds the tissue. Then, the free end 2.2 of each catheter 2 is made to pass through the fastening bayonet 3.2b of the catheter 2, and is firmly fastened relative to the bayonet. Then, the free end 2.2 of the catheter 2 is plugged into the mounting hole 1.6 of the pump 301, to complete the mounting. The skin is seamed after it is tested through the remote control device 4 that the electric urination control device works effectively, to complete an implantation operation.

The tissue compression device in the present invention is applicable to curing a dysfunction of an organ of a man or an animal. For example, the tissue compression device is used to cure urinary incontinence, copracrasia, constipation, impotence, a gall stone, and problems occurring on parts such as a stomach, an intestine, and a hepatic duct, and is suitable for controlling blood flowing in a vessel or ovulation in a female uterus.

When the tissue compression device in the present invention is used to control flowing of food in the stomach of the patient or intestinal content, the compression device 3 softly compresses at least one part of a tissue wall of the stomach or the intestine of the patient, to affect flowing of food in the stomach or the intestinal content.

When the tissue compression device in the present invention is used to control flowing of urine in the tissue or the urinary bladder, the compression device 3 softly compresses the urine flowing in the tissue or the urinary bladder of the patient.

When the tissue compression device in the present invention is used as an impotence curing device, the compression device 3 softly compresses one or two of the penis or the corpus cavernosum of the patient, to limit blood flowing away from the penis, to implement hardening of the penis.

When the tissue compression device in the present invention is used to control blood flowing in the vessel of the patient, the compression device 3 softly compresses at least one part of a tissue wall of the vessel of the patient, to control blood flowing in the vessel.

When the tissue compression device in the present invention is used to control an ovum of the patient to flow to the female uterus, the compression device 3 softly compresses each female fallopian tube to limit a path thereof, and release the fallopian tube to allow an ovum in the path of the fallopian tube to enter a uterine cavity.

When the tissue compression device in the present invention is used to control flowing of a gall stone of the patient, the compression device 3 compresses a part of a tissue wall of the gall bladder, the hepatic duct, or the bile duct, so that the tissue wall partially contracts, and one or more gall stones in the duct move towards the duodenum.

In the prior art, a solution used to prevent tissue degeneration resulting from poor blood circulation is: Two or more compression elements that are separately operated are separately arranged on a tissue wall of an organ, and the elements are sequentially operated, so that each tissue wall has enough time to recover, that is, recover to normal blood circulation, and meanwhile, one of the other tissue walls is compressed. However, a device designed based on the solution has a plurality of disadvantages. First, this device occupies large space, therefore, it cannot be implanted actually. Second, this device is operated, so that the compression elements are moved between a compressed location and a non-compressed location day and night, and this needs a large amount of supplies of power. The large amount of supplies of power makes it necessary to implant a high-capacity battery and/or a complex system that is used to transmit wireless energy constantly from the outside of the patient to frequently charge an implanted rechargeable battery. In this way, this device is unpractical or even unreal due to a large size and high power consumption. Third, a complex control system is needed to control a moving element. Finally, the foregoing type of complex device greatly increases costs of curing a tissue dysfunction. The tissue compression device in the present invention has a simple structure, occupies small space, is easy to be implanted, and has low power consumption and a simple operation.

In the present invention, the compression device 3 of the tissue compression device includes at least three hollow compression elements, and the at least three compression elements are nonlinearly arranged along an axial direction of the tissue. It is a virtue of such arrangement that can avoid 360° circumferentially compressing exerted by a tissue constraint mechanism in the prior art to a tissue, therefore, ischemic necrosis will very easily occur on the tissue and bring serious complication.

The connection line of the center points of any two compression elements is not parallel with the axis of the tissue, to avoid that two compression elements repeatedly compress a same part of the tissue at the same time.

The compression elements do not act on a same cross section that is perpendicular to the axis of the tissue. In other words, a cross section whereon at least one compression element is perpendicular to the axis and is not on a same plane with the cross sections whereon the other compression elements are perpendicular to the axis, therefore, pressure exerted on the tissue is further dispersed, and pressure from different directions is prevented from acting on a same segment of the tissue.

The compression elements are disposed on the substrate that basically has the arc-shaped contour. The compression elements are distributed on the substrate, so that the arc-shaped substrate can stably surround the tissue. The substrate bent to the arc shape does not directly compress the tissue, and the compression elements fastened to the substrate compress the tissue only when expanding under pressure, to control a flow of urine in the tissue, and avoid ischemic necrosis of the tissue.

The power supply, the pump 301, and the liquid storage chamber 1.5 are of an integral structure, and the piston is connected to the pump body 1.2 via the spring 1.4. The piston adjusts the volume of the liquid storage chamber 1.5 through reciprocating movement. The spring 1.4 at least partially drives the piston to adjust the volume of the liquid storage chamber 1.5. The pump 301 pumps water out when driven by a motor, and pumps water back under tensile force of the spring 1.4. Therefore, there is no other energy consumption, to fully play a role of the power supply.

To sum up, the tissue compression device in the present invention has a simple structure, occupies small space, is easy to be implanted, and has low power consumption and a simple operation.

In the present invention, the compression device of the tissue compression device nonlinearly compresses the tissue wall relative to the axis of the tissue. For example, at least three hollow compression elements are disposed on the compression device, and the at least three compression elements are nonlinearly arranged along the axial direction of the tissue. It is a virtue of such arrangement that can avoid 360° circumferentially compressing exerted by a tissue constraint mechanism in the prior art to a tissue, therefore, ischemic necrosis will very easily occur on the tissue and bring serious complication.

The connection line of the center points of any two compression elements is not parallel with the axis of the tissue, to avoid that two compression elements repeatedly compress a same part of the tissue at the same time.

The compression elements do not act on a same cross section that is perpendicular to the axis of the tissue. In other words, a cross section whereon at least one compression element is perpendicular to the axis and is not on a same plane with the cross sections whereon the other compression elements are perpendicular to the axis, therefore, pressure exerted on the tissue is further dispersed, and pressure from different directions is prevented from acting on a same segment of the tissue.

The compression elements are disposed on the substrate that basically has the arc-shaped contour. The compression elements are distributed on the substrate, so that the arc-shaped substrate can stably surround the tissue. The substrate bent to the arc shape does not directly compress the tissue, and the compression elements fastened to the substrate compress the tissue only when expanding under pressure, to control a flow of urine in the tissue, and avoid ischemic necrosis of the tissue.

The power supply, the pump, and the liquid storage chamber are of an integral structure, and the pump head is connected to the pump body via the spring. The pump head adjusts the volume of the liquid storage chamber through reciprocating movement. The spring at least partially drives the pump head to adjust the volume of the liquid storage chamber. The pump 301 pumps water out when driven by a motor, and pumps water back under tensile force of the spring, or under the action of a piston, or under the cooperated action of the spring and the piston, to reduce power consumption to some extent, and improve efficiency.

Preferably, the tissue compression device further includes the sensor, where the sensor is used to sense a physiological parameter of the inside of the patient's body and/or a functional parameter of the tissue compression device, and send the physiological parameter and/or the functional parameter to the control device, and the control device adjusts delivery of the fluid based on the physiological parameter and/or the functional parameter. The tissue compression device is in a powered-on and standby state. When the physiological parameter of the inside of the patient's body and/or the functional parameter of the tissue compression device exceeds a threshold or starts to increase, for example, the patient coughs or sneezes, the sensor sends a signal to the controller, and the compression device rapidly compresses the tissue, to avoid a phenomenon of liquid leakage of the tissue when the patient coughs or sneezes.

It should be finally noted that the foregoing embodiments are merely intended for describing the technical solutions of the present invention, but not for limiting the present invention. Although the present invention is described in detail with reference to the foregoing embodiments, persons of ordinary skill in the art should understand that they may still make modifications to the technical solutions recorded in the foregoing embodiments or make equivalent replacements to some technical features thereof, without departing from the spirit and scope of the technical solutions of the embodiments of the present invention.

## Claims

1. A tissue compression device, comprising a pump, a liquid storage chamber, catheters, and a compression device, wherein the liquid storage chamber is coupled to the pump, the liquid storage chamber stores fluid of a preset volume, the liquid storage chamber communicates with the compression device via the catheters, the pump provides power for fluid delivery between the liquid storage chamber and the compression device, and the compression device is used to nonlinearly compress a tissue wall relative to an axis of a tissue.

2. The tissue compression device according to claim 1, further comprising a power supply, wherein the power supply is coupled to the pump, and supplies electric energy to the pump.

3. The tissue compression device according to claim 2, wherein the pump delivers, driven by the electric energy, the fluid in the liquid storage chamber to the compression device via a catheter, and delivers, driven by mechanical energy, the fluid back to the liquid storage chamber via a catheter.

4. The tissue compression device according to claim 3, wherein the pump comprises a pump body, a pump head, and a spring, the pump head is connected to the pump body via the spring, the pump head adjusts a volume of the liquid storage chamber through reciprocating movement, and the spring at least partially drives the pump head to adjust the volume of the liquid storage chamber.

5. The tissue compression device according to claim 4, wherein the pump head comprises a piston, the spring is coupled to the piston, the piston is coupled to an inner wall of the liquid storage chamber, and at least one of the piston and the spring delivers, driven by non-electric energy, the fluid back to the liquid storage chamber.

6. The tissue compression device according to any one of claims 1 to 5, wherein the compression device further comprises at least one hollow flexible substrate, compression elements are disposed on the substrate, and the compression elements internally communicate with the substrate.

7. The tissue compression device according to claim 6, wherein the substrate has a closed or non-closed curved surface.

8. The tissue compression device according to claim 7, wherein the closed or non-closed curved surface comprises a concave surface, and the concave surface fits the tissue.

9. The tissue compression device according to claim 7, wherein a fastening piece is disposed on the substrate, and is used to fasten the substrate to the peripheral of the tissue.

10. The tissue compression device according to claim 9, wherein the fastening piece comprises a clasp, and the clasp can be coupled to the catheter.

11. The tissue compression device according to claim 9, wherein the fastening piece comprises a clasp and a cartridge, and the clasp fits the cartridge, to fasten the substrate to the peripheral of the tissue.

12. The tissue compression device according to claim 1, wherein when the fluid in the liquid storage chamber is introduced to the compression device, the compression device nonlinearly compresses the tissue wall relative to the axis of the tissue, and when the fluid flows back to the liquid storage chamber, the compression device does not compress the tissue wall.

13. The tissue compression device according to claim 1, wherein the compression device comprises at least three hollow compression elements, and the at least three compression elements are nonlinearly arranged along an axial direction of the tissue.

14. The tissue compression device according to claim 13, wherein a connection line of center points of any two compression elements is not parallel with the axis of the tissue.

15. The tissue compression device according to claim 14, wherein cross sections whereon the center points of any two compression elements are perpendicular to the axis of the tissue are not on a same plane.

16. The tissue compression device according to any one of claims 13 to 15, wherein the compression elements each have an arc-shaped contour, and the arc-shaped contour spirally contracts towards the axis of the tissue.

17. The tissue compression device according to claim 1, wherein there are two catheters, one end of each catheter is coupled to a substrate, and the other end is coupled to the pump.

18. The tissue compression device according to claim 1 or 17, wherein a valve is disposed on each of the catheters, and the valve is used to change a flow direction of the fluid.

19. The tissue compression device according to claim 18, wherein the valve is a one-way valve, to control the fluid to flow in one way.

20. The tissue compression device according to claim 1 or 17, wherein a free end is disposed on each catheter, and the free end of the catheter can be integrated with or separated from at least one of the tissue compression device and the pump.

21. The tissue compression device according to claim 20, wherein a first coupling piece is disposed on the free end of the catheter, a second coupling piece is disposed on the pump, and the first coupling piece is in interference fit with the second coupling piece.

22. The tissue compression device according to claim 21, wherein the first coupling piece is a mounting ratchet, and the second coupling piece is a mounting hole.

23. The tissue compression device according to any one of claims 1 to 5, further comprising a control device, wherein the control device is used to control working of the pump.

24. The tissue compression device according to claim 23, wherein a patient controls the control device in vitro.

25. The tissue compression device according to claim 23, further comprising a sensor, wherein the sensor is used to sense a physiological parameter of the inside of the patient' body and/or a functional parameter of the tissue compression device, and send the physiological parameter and/or the functional parameter to the control device, and the control device adjusts delivery of the fluid based on the physiological parameter and/or the functional parameter.

26. The tissue compression device according to claim 25, wherein the control device is an in vitro wireless remote control device.

27. The tissue compression device according to claim 25 or 26, wherein the physiological parameter is pressure inside the patient' body, and the functional parameter is pressure of the compression device.

28. The tissue compression device according to claim 23, wherein the control device is a limit switch, and the limit switch is connected in series to the pump, and is used to limit stroke of the pump.

29. The tissue compression device according to claim 28, wherein a patient controls the control device in vitro.

30. The tissue compression device according to claim 29, wherein the control device comprises an in vitro remote control device and an in vivo sensing device, and the in vivo sensing device is used to implement wireless coupling to the in vitro wireless remote control device, and control the compression device based on a coupling signal.

31. The tissue compression device according to any one of claims 28 to 30, further comprising a sensor, wherein the sensor is used to sense a physiological parameter of the inside of the patient's body and/or a functional parameter of the tissue compression device, and send the physiological parameter and/or the functional parameter to the control device, and the control device adjusts delivery of the fluid based on the physiological parameter and/or the functional parameter.

32. The tissue compression device according to claim 31, wherein the control device is an in vitro wireless remote control device.

33. The tissue compression device according to claim 1, wherein the tissue is selected from any one of a group comprising a penis, a urethra, a bladder, a blood vessel, a stomach, an intestine, a fallopian tube, a gall bladder, a hepatic duct, and a bile duct.
